# EUROPEAN PATENT SPECIFICATION

(11) **EP 2 808 321 B1**
(45) Date of publication and mention of the grant of the patent: **01.12.2021**
(21) Application number: 13741649.1
(22) Date of filing: 25.01.2013
(51) Int. Cl.: C07C 319/02, C07C 321/04, G02B 3/00, C08G 18/52

(54) **METHOD FOR PRODUCING POLYTHIOL COMPOUND FOR OPTICAL MATERIALS AND COMPOSITION COMPRISING SAME FOR OPTICAL MATERIALS**
VERFAHREN ZUR HERSTELLUNG EINER POLYTHIOLVERBINDUNG FÜR OPTISCHE MATERIALIEN UND DIESE ENTHALTENDE ZUSAMMENSETZUNGEN FÜR OPTISCHE MATERIALIEN
PROCÉDÉ DE PRODUCTION D'UN COMPOSÉ POLYTHIOL POUR MATÉRIAUX OPTIQUES ET COMPOSITION COMPRENANT CELUI-CI POUR MATÉRIAUX OPTIQUES

(30) Priority: 25.01.2012 KR 20120007441
(43) Date of publication of application: 03.12.2014
(73) Proprietor: KOC Solution Co. Ltd., Daejeon 305-380 (KR)
(72) Inventor: JANG, Dong Gyu, Daejeon 302-777 (KR); ROH, Soo Gyun, Daejeon 305-790 (KR); KIM, Jong Hyo, Daejeon 305-251 (KR)
(74) Representative: Serjeants LLP
(86) International application number: PCT/KR2013/000626
(87) International publication number: WO 2013/111999

(56) References cited:
- EP-A1- 2 011 785
- JP-A- H05 208 950
- JP-A- H09 286 772
- KR-A- 20080 090 529
- KR-B1- 920 005 917
- US-A- 5 169 964
- US-B1- 6 288 248
- "EPICHLOROHYDRIN PRODUCT DATA SHEET", SOLVAY CHEMICALS INTERNATIONAL,, no. 7, 1 July 2011 (2011-07-01), page 1, XP003035821,
- None

## Description

### Technical Field

The present invention relates to a method for preparing a polythiol compound for an optical material and a composition for an optical material including a polythiol compound prepared by the method. More particularly, the present invention relates to a polythiol compound for a high quality optical material that exhibits excellent physical properties in terms of color, a composition for an optical material including the polythiol compound, and a method for producing an optical material.

### Background Art

Plastic optical materials are used for optical lenses such as spectacle lenses. Such plastic optical materials are more lightweight, less brittle, and more easily dyeable than optical materials composed of inorganic materials. In recent years, numerous plastic resin materials have been used as optical materials and have been increasingly required to have excellent physical properties.

Korean Patent Publication Nos. 1993-0006918 and 1992-0005708 propose thiourethane lenses manufactured by reacting polythiol compounds with polyisocyanate compounds. Polythiourethane optical materials produced using polythiol compounds and isocyanate compounds are widely used as optical lens materials due to their excellent optical properties in terms of transparency, Abbe number and transmittance, and excellent physical properties in terms of tensile strength. In some cases, however, the use of colored polythiol compounds for polymerization may worsen the color of resins to be produced. When it is intended to produce less colored, transparent resins with good color characteristics by polymerization of polythiol compounds, the coloring of the polythiol compounds should be suppressed as much as possible.

European Patent Application No. 2011785 (corresponding to Korean Patent Publication No. 10-2008-0090529) reveals that poor color characteristics of a polyurethane resin are attributed to the presence of bis(2-hydroxyethyl)disulfide as an impurity in 2-mercaptoethanol, which is a raw material in the preparation of a polythiol. This patent publication describes a method for preparing a polythiol compound by reacting 2-mercaptoethanol with an epihalohydrin compound wherein the 2-mercaptoethanol contains 0.5% by weight or less of bis(2-hydroxyethyl)disulfide.

### Disclosure of the Invention

### Technical Problem

It is known that the presence of bis(2-hydroxyethyl)disulfide as an impurity in 2-mercaptoethanol is a factor affecting the coloring of polythiol compounds. However, even in the case where this factor is controlled, some polythiol compounds are still colored. In view of this situation, the present inventors have conducted research on other factors affecting the coloring of polythiol compounds, and as a result, found that the content of particular impurities in epichlorohydrin compounds as starting materials in the synthesis of polythiol compounds has a direct influence on the coloring of the polythiol compounds.

It is an object of the present invention to provide a method for preparing a less colored polythiol compound by controlling the content of particular impurities in an epichlorohydrin compound, a composition for an optical material including a polythiol compound prepared by the method, and a method for preparing an optical material.

### Technical Solution

The present inventors have found that when the total content of particular impurities in an epichlorohydrin compound as a starting material in the synthesis of a polythiol compound is not greater than a predetermined value, the coloring of the polythiol compound can be minimized and the polythiol compound can be polymerized to produce a urethane resin that is prevented from being colored, has a low yellowness index and is good in terms of color.

The present invention provides a method for preparing a polythiol compound according to claim 1.

The present invention also provides a method for producing a urethane optical material comprising cast polymerizing a composition comprising a polythiol compound containing 1,2-bis[(2-mercaptoethyl)thio]-3-mercaptopropane prepared by the method according to claim 1, and a polyisocyanate compound.

### Advantages Effects

According to the method of the present invention, the content of particular impurities in an epichlorohydrin compound as a starting material in the synthesis of a polythiol compound is controlled below a predetermined level, so that the polythiol compound can be prevented from being colored. The use of the polythiol compound enables the production of a urethane optical material that is prevented from being colored and has a low yellowness index and a good color.

### Best Mode

The present invention provides a method for preparing a polythiol compound containing 1,2-bis[(2-mercaptoethyl)thio]-3-mercaptopropane. The method of the present invention is known in the art. Specifically, the method of the present invention includes reacting an epichlorohydrin compound with 2-mercaptoethanol to obtain a polyalcohol compound, reacting the polyalcohol compound with thiourea to obtain a thiouronium salt, and hydrolyzing the thiouronium salt to obtain a polythiol compound containing 1,2-bis[(2-mercaptoethyl)thio]-3-mercaptopropane. According to the method of the present invention, the content of particular impurities in an epichlorohydrin compound as a starting material is controlled below a predetermined level to obtain a polythiol compound that is prevented from being colored. The impurities in the epichlorohydrin compound include acrolein, allyl chloride, 1,2-dichloropropane, 2,3-dichloropropene, 2-methyl-2-pentanol, 2-chloroallyl alcohol, cis-1,3-dichloropropene, trans-1,3-dichloropropene, 1,3-dichloroisopropanol, 1,2,3-trichloropropane and 2,3-dichloropropanol. When the total content of the impurities is limited to 0.5% by weight or less, based on the weight of the epichlorohydrin compound, a polythiol compound that is prevented from being colored and has a good color can be obtained.

The present invention provides a method for producing a urethane optical material comprising cast polymerizing a composition comprising a polythiol compound containing 1,2-bis[(2-mercaptoethyl)thio]-3-mercaptopropane prepared by the above method, and a polyisocyanate compound.

The polyisocyanate compound used in the method of the present invention may be any compound having at least one isocyanate group and/or at least one isothiocyanate group. Examples of such polyisocyanate compounds include: aliphatic isocyanate compounds, such as 2,2-dimethylpentane diisocyanate, hexamethylene diisocyanate, 2,2,4-trimethylhexane diisocyanate, butene diisocyanate, 1,3-butadiene-1,4-diisocyanate, 2,4,4-trimethylhexamethylene diisocyanate, 1,6,11-undecane triisocyanate, 1,3,6-hexamethylene triisocyanate, 1,8-diisocyanato-4-isocyanatomethyloctane, bis(isocyanatoethyl)carbonate, and bis(isocyanatoethyl)ether; alicyclic isocyanate compounds, such as isophorone diisocyanate, 1,2-bis(isocyanatomethyl)cyclohexane, 1,3-bis(isocyanatomethyl)cyclohexane, 1,4-bis(isocyanatomethyl)cyclohexane, dicyclohexylmethane diisocyanate, cyclohexane diisocyanate, methylcyclohexane diisocyanate, dicyclohexyldimethylmethane isocyanate, and 2,2-dimethyldicyclohexylmethane isocyanate; aromatic isocyanate compounds, such as xylylene diisocyanate (XDI), bis(isocyanatoethyl)benzene, bis(isocyanatopropyl)benzene, bis(isocyanatobutyl)benzene, bis(isocyanatomethyl)naphthalene, bis(isocyanatomethyl)diphenyl ether, phenylene diisocyanate, ethylphenylene diisocyanate, isopropylphenylene diisocyanate, dimethylphenylene diisocyanate, diethylphenylene diisocyanate, diisopropylphenylene diisocyanate, trimethylbenzene triisocyanate, benzene triisocyanate, biphenyl diisocyanate, toluidine diisocyanate, 4,4-diphenylmethane diisocyanate, 3,3-dimethyldiphenylmethane-4,4-diisocyanate, bibenzyl-4,4-diisocyanate, bis(isocyanatophenyl)ethylene, 3,3-dimethoxybiphenyl-4,4-diisocyanate, hexahydrobenzene diisocyanate, and hexahydrodiphenylmethane-4,4-diisocyanate; sulfur-containing aliphatic isocyanate compounds, such as bis(isocyanatoethyl)sulfide, bis(isocyanatopropyl)sulfide, bis(isocyanatohexyl)sulfide, bis(isocyanatomethyl)sulfone, bis(isocyanatomethyl)disulfide, bis(isocyanatopropyl)disulfide, bis(isocyanatomethylthio)methane, bis(isocyanatoethylthio)methane, bis(isocyanatoethylthio)ethane, bis(isocyanatomethylthio)ethane, and 1,5-diisocyanato-2-isocyanatomethyl-3-thiapentane; sulfur-containing aromatic isocyanate compounds, such as diphenylsulfide-2,4-diisocyanate, diphenylsulfide-4,4-diisocyanate, 3,3-dimethoxy-4,4-diisocyanatodibenzyl thioether, bis(4-isocyanatomethylbenzene)sulfide, 4,4-methoxybenzenethioethylene glycol-3,3-diisocyanate, diphenyldisulfide-4,4-diisocyanate, 2,2-dimethyldiphenyldisulfide-5,5-diisocyanate, 3,3-dimethyldiphenyldisulfide-5,5-diisocyanate, 3,3-dimethyldiphenyldisulfide-6,6-diisocyanate, 4,4-dimethyldiphenyldisulfide-5,5-diisocyanate, 3,3-dimethoxydiphenyldisulfide-4,4-diisocyanate, and 4,4-dimethoxydiphenyldisulfide-3,3-diisocyanate; and sulfur-containing heterocyclic isocyanate compounds, such as 2,5-diisocyanatothiophene, 2,5-bis(isocyanatomethyl)thiophene, 2,5-diisocyanatotetrahydrothiophene, 2,5-bis(isocyanatomethyl)tetrahydrothiophene, 3,4-bis(isocyanatomethyl)tetrahydrothiophene, 2,5-diisocyanato-1,4-dithiane, 2,5-bis(isocyanatomethyl)-1,4-dithiane, 4,5-diisocyanato-1,3-dithiolane, 4,5-bis(isocyanatomethyl)-1,3-dithiolane, and 4,5-bis(isocyanatomethyl)-2-methyl-1,3-dithiolane. These polyisocyanate compounds may be used alone or as a mixture of two or more thereof. Other compounds having at least one isocyanate group and/or at least one isothiocyanate group may be used alone or as a mixture of two or more thereof. Halogenated products (for example, chlorinated and brominated products) of the isocyanate compounds may also be used. Alkylated products, alkoxylated products and nitro-substituted products of the isocyanate compounds may also be used. Prepolymer modified products of the isocyanate compounds with polyhydric alcohols or thiols may also be used. Carbodiimide-, urea- and biuret-modified products of the isocyanate compounds may also be used. Dimerization or trimerization reaction products of the isocyanate compounds may also be used.

The polyisocyanate compound is preferably selected from isophorone diisocyanate (IPDI), hexamethylene diisocyanate (HDI), dicyclohexyl methanediisocyanate (H12MDI), xylylene diisocyanate (XDI), 3,8-bis(isocyanatomethyl)tricyclo[5,2,1,0^{2,6}]decane, 3,9-bis(isocyanatomethyl)tricyclo[5,2,1,0^{2,6}]decane, 4,8-bis(isocyanatomethyl)tricyclo[5,2,1,0^{2,6}]decane, 2,5-bis(isocyanatomethyl)bicyclo[2,2,1]heptane, 2,6-bis(isocyanatomethyl)bicyclo[2,2,1]heptane, and mixtures thereof.

The urethane optical material has the advantages of high refractive index, low dispersity, good heat resistance, superior durability, light weight, and good impact resistance. Particularly, the optical material is clear and transparent due to its good color. Due to these advantages, the urethane resin of the present invention is suitable for use in optical products such as lenses and prisms, particularly lenses such as spectacle lenses and camera lenses.

### EXAMPLES

The present invention will be explained in more detail with reference to the following examples. However, these examples are provided for illustrative purposes only and are not intended to limit the scope of the present invention.

### Evaluation Methods

Compounds were analyzed by the following methods. The colors of polythiol compounds and resins were evaluated based on YI and APHA values thereof, which were measured by the following test methods.

Gas chromatography, gas chromatography-mass spectrometry (GC-MS), and NMR spectroscopy were used to measure the content of epichlorohydrin and analyze impurities in the epichlorohydrin.

The colors (YI or dYI) of polythiol compounds were measured using a UV-Vis spectrophotometer (Model UV-2450, SHIMADZU) fitted with an IRS-2200 condenser. Distilled water was placed in two silica cells having the same length (1 cm), and then a base line was determined. After addition of each polythiol compound to one of the silica cells, the YI value of the polythiol compound was measured. YI represents yellowness index and can be measured using a colorimeter. A smaller YI value indicates a better color.

The colors (YI or dYI) of polythiourethane plastic lenses were measured using a UV-Vis spectrophotometer (Model UV-2450, SHIMADZU) fitted with an IRS-2200 condenser. For the measurement, the YI of air was used as the reference and each lens was fixed to a lens clamp. YI represents yellowness index and can be measured using a colorimeter. A lower YI indicates a better color.

The APHA values of polythiol compounds were measured using a spectrophotometer (ColorQuest XE, Hunterlab). After each sample was placed in a quartz cell having a path length of 1 cm, the APHA value of the sample was measured by comparison of a program containing data on the concentrations of reference solutions of platinum and cobalt reagents with the sample solution. The smaller the measured value, the better is the color.

The APHA values of polythiourethane plastic lenses were measured using a spectrophotometer (ColorQuest XE, Hunterlab). After each plastic lens was placed in the instrument, the APHA value of the lens was measured by comparison of a program containing data on the concentrations of reference solutions of platinum and cobalt reagents with the sample solution. The smaller the measured value, the better is the color.

### Example 1

### Synthesis of 1,2-bis[(2-mercaptoethyl)thio]-3-mercaptopropane

676 g (8.65 mol) of 2-mercaptoethanol having purity ≥ 99.9% and 340 g of water were put into a 10-liter five-neck reaction flask equipped with a stirrer, a reflux condenser, a nitrogen purging tube, and a thermometer. 691.2 g (1.08 mol) of an aqueous solution of 25 wt% sodium hydroxide was added dropwise to the flask at 30 °C over 30 min, and then 399.6 g (4.32 mol) of epichlorohydrin was added dropwise thereto at the same temperature over 3 hr. The mixture was allowed to stand for 1 hr. The total content of impurities including acrolein, allyl chloride, 1,2-dichloropropane, 2,3-dichloropropene, 2-methyl-2-pentanol, 2-chloroallyl alcohol, cis-1,3-dichloropropene, trans-1,3-dichloropropene, 1,3-dichloroisopropanol, 1,2,3-trichloropropane and 2,3-dichloropropanol in the epichlorohydrin was not greater than 0.1%. That is, the purity of the epichlorohydrin was ≥ 99.9%. Then, 1800 g (17.28 mol) of 35 wt% hydrochloric acid and 987.6 g (12.97 mol) of thiourea were added, and the resulting mixture was heated to reflux at 110 °C for 3 hr to obtain thiouronium salts. After cooling to 20 °C, 1880 g of toluene and 1324.4 g (19.47 mol) of an aqueous solution of 25 wt% ammonia were added to hydrolyze the thiouronium salts. The hydrolysis afforded polythiols containing 1,2-bis[(2-mercaptoethyl)thio]-3-mercaptopropane as a major component. The toluene solution of the polythiols was cleaned with an acid, washed with water, and heated under reduced pressure to remove the toluene and the slight amount of water. 1074.8 g of the polythiols were collected by filtration. The APHA and YI values of the polythiols were 7 and 0.75, respectively.

### Manufacture of plastic lens

52 g of m-xylylene diisocyanate, 0.015 g of dibutyltin dichloride as a curing catalyst, 0.10 g of Zelec UN^{™} (Stepan) as an acidic phosphate, and 0.05 g of Viosorb 583 as a UV absorber were mixed and dissolved at 20 °C. The solution was mixed with 48 g of the polythiols containing 1,2-bis[(2-mercaptoethyl)thio]-3-mercaptopropane as a major component to prepare a homogeneous solution. The homogeneous solution was degassed at 400 Pa for 1 h, filtered through a 1 µm PTFE filter, and filled in a mold composed of a glass mold and a tape. After the mold was charged into a polymerization oven, the temperature was slowly raised from 10 to 120 °C over 20 h. After completion of the polymerization, the mold was taken out of the oven and the lens was released therefrom. The lens was further annealed at 120 °C for 3 hr. The resin had an APHA value of 10 and a YI value of 0.9. The results are shown in Table 1.

### Example 2

Polythiols containing 1,2-bis[(2-mercaptoethyl)thio]-3-mercaptopropane as a major component were synthesized in the same manner as in Example 1, except that epichlorohydrin containing impurities in a total amount of 0.05 wt% was used. The polythiols were measured to have an APHA value of 8 and a YI value of 0.81. A plastic lens was manufactured using the polythiols in the same manner as in Example 1. The results of evaluations are shown in Table 1.

### Example 3

Polythiols containing 1,2-bis[(2-mercaptoethyl)thio]-3-mercaptopropane as a major component were synthesized in the same manner as in Example 1, except that epichlorohydrin containing impurities in a total amount of 0.1 wt% was used. The polythiols were measured to have an APHA value of 11 and a YI value of 0.92. A plastic lens was manufactured using the polythiols in the same manner as in Example 1. The results of evaluations are shown in Table 1.

### Example 4

Polythiols containing 1,2-bis[(2-mercaptoethyl)thio]-3-mercaptopropane as a major component were synthesized in the same manner as in Example 1, except that epichlorohydrin containing impurities in a total amount of 0.23 wt% was used. The polythiols were measured to have an APHA value of 13 and a YI value of 1.12. A plastic lens was manufactured using the polythiols in the same manner as in Example 1. The results of evaluations are shown in Table 1.

### Example 5

Polythiols containing 1,2-bis[(2-mercaptoethyl)thio]-3-mercaptopropane as a major component were synthesized in the same manner as in Example 1, except that epichlorohydrin containing impurities in a total amount of 0.4 wt% was used. The polythiols were measured to have an APHA value of 16 and a YI value of 1.23. A plastic lens was manufactured using the polythiols in the same manner as in Example 1. The results of evaluations are shown in Table 1.

### Comparative Example 1

Polythiols containing 1,2-bis[(2-mercaptoethyl)thio]-3-mercaptopropane as a major component were synthesized in the same manner as in Example 1, except that epichlorohydrin containing impurities in a total amount of 0.8 wt% was used. The polythiols were measured to have an APHA value of 26 and a YI value of 1.87. A plastic lens was manufactured using the polythiols in the same manner as in Example 1. The results of evaluations are shown in Table 1.

### Comparative Example 2

Polythiols containing 1,2-bis[(2-mercaptoethyl)thio]-3-mercaptopropane as a major component were synthesized in the same manner as in Example 1, except that epichlorohydrin containing impurities in a total amount of 1.3 wt% was used. The polythiols were measured to have an APHA value of 30 and a YI value of 2.36. A plastic lens was manufactured using the polythiols in the same manner as in Example 1. The results of evaluations are shown in Table 1.

### Comparative Example 3

Polythiols containing 1,2-bis[(2-mercaptoethyl)thio]-3-mercaptopropane as a major component were synthesized in the same manner as in Example 1, except that epichlorohydrin containing impurities in a total amount of 1.8 wt% was used. The polythiols were measured to have an APHA value of 38 and a YI value of 2.53. A plastic lens was manufactured using the polythiols in the same manner as in Example 1. The results of evaluations are shown in Table 1.

**TABLE 1**

| Example No. | Total content of impurities in epichlorohydrin (%)^{∗} | Color of polythiol | | Color of plastic lens | |
|---|---|---|---|---|---|
| | | APHA | YI | APHA | YI |
| Example 1 | 0.01 | 7 | 0.75 | 10 | 0.9 |
| Example 2 | 0.05 | 8 | 0.81 | 13 | 0.9 |
| Example 3 | 0.1 | 11 | 0.92 | 16 | 1.1 |
| Example 4 | 0.23 | 13 | 1.12 | 18 | 1.2 |
| Example 5 | 0.4 | 16 | 1.23 | 19 | 1.4 |
| Comparative Example 1 | 0.8 | 26 | 1.87 | 40 | 3.1 |
| Comparative Example 2 | 1.3 | 30 | 2.36 | 43 | 3.3 |
| Comparative Example 3 | 1.8 | 38 | 2.53 | 44 | 3.4 |

| | | | | | |
|---|---|---|---|---|---|
| ^{∗} Total content of impurities including acrolein, allyl chloride, 1,2-dichloropropane, 2,3-dichloropropene, 2-methyl-2-pentanol, 2-chloroallyl alcohol, cis-1,3-dichloropropene, trans-1,3-dichloropropene, 1,3-dichloroisopropanol, 1,2,3-trichloropropane and 2,3-dichloropropanol | | | | | |

As can be seen from the results in Table 1, the resins of Comparative Examples 1-3 were more yellow in color than the resins of Examples 1-5.

Despite the use of highly pure 2-mercaptoethanol as a raw material, the presence of impurities above a predetermined level in epichlorohydrin worsened the colors of the polythiol compounds in proportion with the content of the impurities, resulting in a worsening in the color of the resins. Particularly, the use of epichlorohydrin containing impurities in a total amount exceeding 0.5 wt% led to a worsening in the color of the polythiols and resins. These results demonstrate that the use of epichlorohydrin containing impurities in a total amount of 0.5 wt% or less enables the production of polythiol compounds and polyurethane resins that are prevented from being colored and have good colors.

### Industrial Applicability

The method of the present invention enables the preparation of a polythiol compound for an optical material that is prevented from being colored. In addition, the polythiol compound can be used to produce an optical material that is prevented from being colored and has a low yellowness index and a good color. The polythiol compound can be used to produce various optical materials such as urethane optical materials. The optical material can be used to manufacture an optical lens having a good color. The optical lens can be widely used as a replacement for conventional optical lenses in various fields. Particularly, the optical lens can be used as a spectacle lens, a polarizing lens, a camera lens, or the like.

## Claims

1. A method for preparing a polythiol compound containing 1,2-bis[(2-mercaptoethyl)thio]-3-mercaptopropane, the method comprising the steps of:
reacting an epichlorohydrin compound with 2-mercaptoethanol to obtain a polyalcohol compound;
reacting the polyalcohol compound with thiourea to obtain a thiouronium salt; and
hydrolyzing the thiouronium salt to obtain a polythiol compound containing 1,2-bis[(2-mercaptoethyl)thio]-3-mercaptopropane;
wherein the epichlorohydrin compound contains 0.5% by weight or less of impurities comprising acrolein, allyl chloride, 1,2-dichloropropane, 2,3-dichloropropene, 2-methyl-2-pentanol, 2-chloroallyl alcohol, cis-1,3-dichloropropene, trans-1,3-dichloropropene, 1,3-dichloroisopropanol, 1,2,3-trichloropropane and 2,3-dichloropropanol.

2. A method for producing a urethane optical material comprising cast polymerizing a composition comprising a polythiol compound containing 1,2-bis[(2-mercaptoethyl)thio]-3-mercaptopropane prepared by the method according to claim 1, and a polyisocyanate compound.

3. The method according to claim 2, wherein the polyisocyanate compound is selected from the group consisting of isophorone diisocyanate (IPDI), hexamethylene diisocyanate (HDI), dicyclohexyl methanediisocyanate (H12MDI), xylylene diisocyanate (XDI), 3,8-bis(isocyanatomethyl)tricyclo[5,2,1,0^{2,6}]decane, 3,9-bis(isocyanatomethyl)tricyclo[5,2,1,0^{2,6}]decane, 4,8-bis(isocyanatomethyl)tricyclo[5,2,1,0²_{'}⁶]decane, 2,5-bis(isocyanatomethyl)bicyclo[2,2,1]heptane, 2,6-bis(isocyanatomethyl)bicyclo[2,2,1]heptane, and mixtures thereof.

## Patentansprüche

1. Verfahren zum Herstellen einer Polythiolverbindung, die 1,2-Bis[(2-mercaptoethyl)thio]-3-mercaptopropan enthält, wobei das Verfahren die folgenden Schritte umfasst:
Umsetzen einer Epichlorhydrinverbindung mit 2-Mercaptoethanol, um eine Polyalkoholverbindung zu erhalten;
Umsetzen der Polyalkoholverbindung mit Thioharnstoff, um ein Thiouroniumsalz zu erhalten; und
Hydrolysieren des Thiouroniumsalzes, um eine Polythiolverbindung zu erhalten, die 1,2-Bis[(2-mercaptoethyl)thio]-3-mercaptopropan enthält;
wobei die Epichlorhydrinverbindung zu 0,5 Gew.-% oder weniger Unreinheiten enthält, umfassend Acrolein, Allylchlorid, 1,2-Dichlorpropan, 2,3-Dichlorpropen, 2-Methyl-2-pentanol, 2-Chlorallylalkohol, cis-1,3-Dichlorpropen, trans-1,3-Dichlorpropen, 1,3-Dichlorisopropanol, 1,2,3-Trichlorpropan und 2,3-Dichlorpropanol.

2. Verfahren zum Herstellen eines optischen Urethanmaterials, umfassend das Gießpolymerisieren einer Zusammensetzung, die eine Polythiolverbindung, die 1,2-Bis[(2-mercaptoethyl)thio]-3-mercaptopropan, hergestellt durch das Verfahren nach Anspruch 1, enthält, und eine Polyisocyanatverbindung umfasst.

3. Verfahren nach Anspruch 2, wobei die Polyisocyanatverbindung ausgewählt ist aus der Gruppe, bestehend aus Isophorondiisocyanat (IPDI), Hexamethylendiisocyanat (HDI), Dicyclohexylmethandiisocyanat (H12MDI), Xyloldiisocyanat (XDI), 3,8-Bis(isocyanatomethyl)tricyclo[5,2,1,0^{2,6}]decan, 3,9-Bis(isocyanatomethyl)tricyclo[5,2,1,0^{2,6}]decan, 4,8-Bis(isocyanatomethyl)tricyclo[5,2,1,0^{2,6}]decan, 2,5-Bis(isocyanatomethyl)bicyclo[2,2,1]heptan, 2,6-Bis(isocyanatomethyl)bicyclo[2,2,1]heptan und Mischungen davon.

## Revendications

1. Procédé de préparation d'un composé de polythiol contenant du 1,2-bis[(2-mercaptoéthyl)thio]-3-mercaptopropane, le procédé comprenant les étapes consistant à :
faire réagir un composé d'épichlorohydrine avec le 2-mercaptoéthanol pour obtenir un composé de polyalcool ;
faire réagir le composé de polyalcool avec de la thiourée pour obtenir un sel de thiouronium ; et
hydrolyser le sel de thiouronium pour obtenir un composé de polythiol contenant du 1,2-bis[(2-mercaptoéthyl)thio]-3-mercaptopropane ;
dans lequel le composé d'épichlorohydrine contenant 0,5 % en poids ou moins d'impuretés comprenant l'acroléine, le chlorure d'allyle, le 1,2-dichloropropane, le 2,3-dichloropropène, le 2-méthyl-2-pentanol, l'alcool 2-chloroallylique, le cis-1,3-dichloropropène, le trans-1,3-dichloropropène, le 1,3-dichloroisopropanol, le 1,2,3-trichloropropane et le 2,3-dichloropropanol.

2. Procédé de production d'un matériau optique à base d'uréthane comprenant la polymérisation par coulée d'une composition comprenant un composé de polythiol contenant du 1,2-bis[(2-mercaptoéthyl)thio]-3-mercaptopropane préparé par le procédé selon la revendication 1, et d'un composé de polyisocyanate.

3. Procédé selon la revendication 2, dans lequel le composé de polyisocyanate est sélectionné dans le groupe consistant en le diisocyanate d'isophorone (IPDI), le diisocyanate d'hexaméthylène (HDI), le méthanediisocyanate de dicyclohexyle (H12MDI), le diisocyanate de xylylène (XDI), le 3,8-bis(isocyanatométhyl)tricyclo[5,2,1,0^{2,6}]décane,
le 3,9-bis(isocyanatométhyl)tricyclo[5,2,1,0^{2,6}]décane,
le 4,8-bis(isocyanatométhyl)tricyclo[5,2,1,0^{2,6}]décane,
le 2,5-bis(isocyanatométhyl)bicyclo[2,2,1]heptane,
le 2,6-bis(isocyanatométhyl)bicyclo[2,2,1]heptane, et les mélanges de ceux-ci.
